# EUROPEAN PATENT APPLICATION

(11) **EP 2 815 745 A1**
(43) Date of publication of application: **24.12.2014**
(21) Application number: 13173258.8
(22) Date of filing: 21.06.2013
(51) Int. Cl.: A61K 9/48

(54) **Soft shell capsule and process for its manufacture**

(71) Applicant: Swiss Caps Rechte und Lizenzen AG, 9533 Kirchberg SG (CH)
(72) Inventor: Sydow, Georg, 78467 Konstanz (DE); Sennhauser, Markus, 9630 Wattwil (CH); Schluetermann, Burkhard, 79280 Au (DE); Prasch, Armin, 79249 Merzhausen (DE); Nehring, Rainer, 4051 Basel (CH)
(74) Representative: Pfenning, Meinig & Partner GbR

(57) **Abstract**

The invention relates to soft shell capsules having a capsule shell comprising high acyl gellan gum, at least one starch and at least one plasticizer. The invention further relates to a method for manufacturing such soft shell capsules.

## Description

The invention relates to soft shell capsules having a capsule shell comprising high acyl gellan gum, at least one starch and at least one plasticizer. The invention further relates to a method for manufacturing such soft shell capsules.

Gelatine is widely and commonly used in various pharmaceutical and non-pharmaceutical applications including e.g. soft gelatine capsules and hard gelatine capsules. Typically, soft capsules are used to encapsulate primarily liquid matrices, e.g. solutions, emulsions or suspensions, for example of nutritional or pharmaceutical active agent(s). These preparations have many advantages over other dosage forms, permitting accurate delivery of a unit dose in an easy-to-swallow, transportable, in certain cases an improved bioavailable and essentially tasteless form.

However, gelatine has many drawbacks, including the cost and continuity of a safe raw material supply. Sometimes animal sources are also rated as undesirable to certain populations, such as vegetarians and those wishing to maintain Kosher or Halal standards. Further, gelatine is prone to cross-linking, caused by ageing or due to reaction with compounds such as aldehydes, which impact its disintegration and dissolution properties negatively, e.g. prolongation.

Gelatine provides good sealing of the capsule at a temperature above or close to the drop point of a formed ribbon, strong enough to withstand the mechanical forces during encapsulation, showing sufficient elasticity to allow formation of a capsule and dissolves easily in any aqueous media.

With the growing concern of Bovine Spongiform Encephilitis (BSE) disease in products derived from cows, many attempts have been made to replace gelatine. However, these approaches have typically failed in that the resultant products had unacceptably different textural and/or functional properties.

A further material which has recently been used to replace gelatine is gellan gum. In the following the term gellan gum refers to the extracellular polysaccharide obtained by the aerobic fermentation of the microorganism *Pseudomonas elodea* in a suitable nutrient medium. Various forms of gellan gum have been described in the art and may be used in the present invention.

EP 1570 843 B1 teaches a method of producing a blend of high and low acyl gellan gums with starch and a plasticizer having similar textural and functional properties compared to gelatine and its use for the preparation of capsules from this blend.

It was therefore an object of the present invention to provide soft shell capsules which disintegrate under simulated gastric conditions (in vitro).

For solving this objective a method for manufacturing the soft shell capsule with the features of claim 1 and a soft shell capsule with the features of claim 9 is provided. The further dependent claims mention preferred embodiments.

According to the present invention, a process for manufacturing a gellan soft shell capsule is provided, wherein
a) at least one starch is mixed with water to provide a homogeneous suspension,
b) at least one plasticizer is mixed with a high acyl gellan gum having more than 40% acetyl and more than 45% glyceryl residual substituents per repeat unit,
c) the suspension of a) is mixed with the mixture of b) providing a mixed suspension,
d) the mixed suspension of c) is heated to a temperature of 96°C to 99°C,
e) the heated suspension is transported to an encapsulation device in which a filling material is encapsulated to provide the soft shell capsules.

According to a further preferred embodiment, after step e) the soft capsules are dried. The drying is preferably performed by the steps of the pre-drying step with an air blower and a drying step in a tumbler device. In order to stabilize the capsules, they are preferably pre-dried at ambient conditions on a ventilated covered conveyer belt. The belt may have a length of approx. 2 m and may be operated at a rotational speed of approx. 0.02m/s. It is preferred that the pre-dried capsules are subsequently transferred via an intermediate tumbler into a conventional tumble dryer where the capsules are dried until they exhibit an appropriate moisture level, e.g. a residual water activity value a_{w}< 30 %. During the course of the drying procedure the capsules may be de-oiled.

According to a further preferred embodiment of the present invention the soft shell capsules may comprise a low acyl gellan having less than 25% acetyl and less than 15% glyceryl residual substituents per repeat unit.

It is preferred to use a rotary die machine as encapsulation device. In such rotary dye cutting tools the encapsulation material is transported into the slit dies which are individually fed by pumps. The gellan melt is formed by the slits into ribbons which are being fed onto rotating casting drums.

The inner surface of the slit dies is preferably teflonized in order to avoid adherence of the melt to the slit die and to guarantee a more homogeneous melt flow inside the slit dies.

The outlet port of the die is a slit and its width is preferably defined by a spacer which is inserted between the two parts of the die. Different thicknesses of the spacers define the slit width, and therefore also the ribbon thickness. The slit dies are preferably mounted close to the surface of the rotating cooling drums at constant height. The distance between slit die and casting drum can be adjusted as well. The ribbons are cooled down on conventional rotating casting drums which preferably operate at temperatures of about 50°C which is higher as for gelatine ribbons.

Preferably, the at least one starch is a native or a modified starch, preferably selected from the group consisting of potato starch, mung bean starch, corn starch, sago starch, tapioca starch, waxy starch, pea starch and its mixtures, wherein the modification can be physically, chemically or by hydrolysis.

The term starch according to the present invention is intended to include all starches derived from any native source, any of which may be suitable for use herein. A native starch as used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from artificial mutations and variations of the above generic composition, which may be produced by known standard methods of mutation breeding, are also suitable herein.

Typical sources for the starches are cereals, tubers, roots, legumes and fruits. The native source can be any variety of corn (maize), pea, potato, sweet potato, banana, barley, wheat, rice, oat, sago, amaranth, tapioca, arrowroot, canna, sorghum, and waxy and high amylose varieties thereof. As used herein, "waxy" is intended to include a starch containing no more than about 10%, particularly no more than about 5%, more particularly no more than about 3%, and most particularly no more than about 1% amylose by weight. As used herein, the term "high amylose" is intended to include a starch containing at least about 40%, particularly at least about 70%, more particularly at least about 80% by weight amylose. As used herein, the term "amylase-containing" is intended to include a starch containing at least about 10% by weight amylose. In one embodiment, suitable starches are those which are amylase containing starches, in another amylose containing starches which are not high amylose.

The starches may be pre-gelatinized using techniques known in the art and disclosed for example in U.S. Patent Nos. 4,465,702, 5,037,929, 5,131,953, and 5,149,799. Also see, Chapter XXII- "Production and Use of Pregelatinized Starch", Starch: Chemistry and Technology, Vol. III-Industrial Aspects, R.L. Whistler and E.F. Paschall, Editors, Academic Press, New York 1967.

The starch may be a native starch, or a modified starch. Modified starch, as used herein, is intended to include starches which have been modified physically, chemically and/or by hydrolysis. Physical modification includes by shearing or thermally-inhibition, for example by the process described in U.S. Patent No. 5,725,676.

The starch may be chemically modified, including without limitation, crosslinked, acetylated, organically esterified, hydroxyethylated, hydroxypropylated, phosphorylated, inorganically esterified, cationic, anionic, nonionic, and zwitterionic, and succinate and substituted succinate derivatives thereof. Such modifications are known in the art, for example in Modified Starches: Properties and Uses, Ed. Wurzburg, CRC Press, Inc., Florida (1986).

The starches may be hydrolyzed, and suitable starches include fluidity or thin-boiling starches prepared by oxidation, acid hydrolysis, enzyme hydrolysis, heat and or acid dextrinization. These processes are well known in the art.

Any starch having suitable properties for use herein may be purified by any method known in the art to remove starch off flavors and colors that are native to the polysaccharide or created during processing. Suitable purification processes for treating starches are disclosed in the family of patents represented by EP 554 818 (Kasica, et al.). Alkali washing techniques, for starches intended for use in either granular or pre-gelatinized form, are also useful and described in the family of patents represented by U.S. 4,477,480 (Seidel) and 10 5,187,272 (Bertalan et al.).

Suitable starches in the present invention include those which are stabilized, including hydroxyalkylated starches such as hydroxypropylated or hydroxyethylated starches, and acetylated starches. Also suitable are dextrinized starches. In one embodiment, these starches will have a low viscosity, with a water fluidity in the range of from about 20 to 90. In another embodiment, the starches will have a water fluidity in the range of about 65 to 85. Water fluidity is known in the art and, as used herein, is measured using a Thomas Rotational Shear-type Viscometer (commercially available from Arthur A. Thomas Co., Philadelphia, PA), standardized at 30°C with a standard oil having a viscosity of 24.73 cps, which oil requires 23.12 ± 0.05 sec for 100 revolutions. Accurate and reproducible measurements of water fluidity are obtained by determining the time which elapses for 100 revolutions at different solids levels depending on the starch's degree of conversion: as conversion increases, the viscosity decreases. The conversion may be by any method known in the art including oxidation, enzyme conversion, acid hydrolysis, heat and/or acid dextrinization.

The blend further includes at least one plasticizer. The plasticizer used will depend in part upon the end use application. At least one plasticizer is preferably selected from the group consisting of glycerol, xylitol, sorbitol, polyglycerol, non-crystallising solutions of sorbitol, glucose, fructose, glucose syrup, sorbitol/sorbitan solutions, propylene glycol, polyethylene glycols with low molecular weight and combinations thereof. It is preferred that the plasticizer has a water content of less than 17.5% (w/w) and more preferably less than 2.5% (w/w).

The soft capsule is preferably filled with the filling material selected from the group consisting of foods, flavourings, vitamins, pharmaceuticals, detergents, liquids, semi-solids, suspensions, cosmetics, bath oils and its mixtures. In a preferred embodiment the filling material is selected from the group consisting of fish oil, krill oil, peppermint oil, eucalyptus oil, garlic oil and garlic oil mazerates, lin seed oil, evening primrose oil, essential oils e.g. alpha-pinen, beta-pinen, anethol, fencheon, cineol, camphen, borneo-campher, mistletoe oil and products and mixtures thereof.

Moreover, the following essential oils can be used as a filling material:
Allspice, aniseed, basil, bay, benzoin, bergamot, black pepper, cajuput, camomile, camphor, caraway, carrot seed, cassia, cedarwood, chamomile, cinnamon, citronella, clary sage, clove, coriander, cypress, dill, eucalyptus, fennel, frankincense, geranium, ginger, grapefruit, helichrysum, hyssop, jasmine, juniper, lavandin, lavender, lemon, lemongrass, lemon verbena, lime, mandarin, marjoram, melissa, myrrh, neroli, niaouli, nutmeg, orange, pamarosa, patchouli, peppermint, petitgrain, pimento, pine, rose, rose geranium, rosemary rosewood, sage, sandalwood, spearmint, tagetes, tangerine, thyme, tea tree, vetiver, ylang-ylang and mixtures thereof.

According to the present invention, a soft shell capsule with a filling material and capsulated in a capsule shell is provided which is producible according to the above described process.

The inventive capsule preferably does not comply with the requirements as defined for capsules with gastric resistant properties, i.e. the capsule is not gastro resistant according to the USP <2040> and disintegrates at least within 120 min, more preferably within 60 min using simulated gastric fluid as the immersion fluid. Furthermore, the capsules may not be gastro resistant according to EP test monograph 2.9.1 using 0.1 M hydrochloric acid as the immersion fluid and capsules disintegrate within 120 min, more preferably within 60 min.

Moreover, it is preferred that the capsule disintegrates according to USP <701> using water or 0.1 M hydrochloric acid or artificial gastric juice R as the immersion fluid within 120 min, preferably within 60 minutes and even more preferably within 30 minutes, or the capsule disintegrates according to EP test monograph 2.9.1 using water or 0.1 M hydrochloric acid or artificial gastric juice R as the immersion fluid within 120 min, preferably within 60 minutes and even more preferably within 30 minutes.

It is further preferred that the capsule ruptures within 15 min according to USP <2040> using water as the immersion fluid.

The inventive capsule has preferably a mean hardness after drying of 2 to 12 N, more preferably 3 to 10 N and even more preferably 4 to 8 N. For the determination of the mean hardness 10 dried capsules have been tested individually. The hardness testing is performed using a hardness tester from Bareiss, e.g. Bareiss hardness tester U73, at a 2 mm push-down level.

With respect to the shell, the composition of the shell, but also the thickness of the shell has to be chosen to guarantee the disintegration of the capsules under gastric testing conditions and the shell material should be minimized to reduce the costs of the capsules. It is therefore preferred that the shell has a thickness after drying of 100 to 900 µm, more preferably 150 to 700 µm and even more preferably 200 to 500 µm.

Furthermore, it is preferred that the shell comprises a first and a second seam, wherein the first seam has a thickness after drying of 50 to 700 µm, preferably 60 to 600 µm and more preferably 100 to 500 µm and the second seam has a thickness after drying of 55 to 650 µm, preferably 65 to 550 µm and more preferably 105 to 505 µm.

Seam and shell thickness can be measured at the final product. Shell and seam thickness ranges are mainly controlled by the ribbon thickness which is to be selected and finally adjusted during the process set-up (s. below).

As one aspect of the present invention the soft capsule may be free of any functional and protective coatings, which are often used for soft capsules according to the prior art.

The capsule shell, as composed during the weighing in procedure for the shell formulation and without the needed purified water used for processing for a soft capsule according to the present invention has preferably the following composition:
a) 5 to 8% (w/w), preferably 6 to 7% (w/w) high acyl gellan gum
b) 0 to 3% (w/w), preferably 0.1 to 2.0% (w/w) low acyl gellan gum
c) 50 to 70% (w/w), preferably 60 to 65% (w/w) of the starch
d) 20 to 40% (w/w), preferably 28 to 32% (w/w) of the plasticizer.

The capsule shell can additionally comprise coloring agents, opacifying agents, antioxidants, preservatives sweeteners, flavoring agents and its mixtures.

The capsule is not limited in view of size and shape. Preferably, the form the soft capsule is spherical, oval or oblong.

The filling materials for the soft capsule shells may be any of those typically used in the art, including oils, hydrophobic or hydrophilic liquids and suspensions and emulsions containing active agents.

The following examples and figures illustrate the present invention without limiting the invention to the specific embodiments mentioned in these examples.

### Example 1

### Description of the manufacturing process

The standard manufacturing process for soft gelatine capsules is the rotary die process. This process is well established in e.g. in the pharmaceutical and nutraceutical industry. In contrast to the standard encapsulation process with gelatine as encapsulation material, the gellan process is to be executed in a closed system at a temperature of minimally 92°C. Below 89°C gellan starts to gel and a process temperature of minimally 92°C prevents gellan from gelling. Initially, the gellan capsule encapsulation material has to be prepared: Therefore, purified water and sodium citrate are combined in a beaker and are being stirred until sodium citrate is completely dissolved. Subsequently, Unipur GA (starch) is added to the solution and is being stirred until a homogeneous suspension is generated. Afterwards the suspension is poured into a tank and is being heated under continuous stirring up to approx. 85-90°C. In between, glycerol is combined with gellan, e.g. Kelcogel LT 100 and Kelcogel F, until a smooth paste is obtained. This preparation is subsequently added to the preheated starch preparation; the temperature is being raised to 98°C.

Other shell excipients like coloring agents, opacifying agents, antioxidants, preservatives, sweeteners, flavouring agents and its mixtures can be added directly to the beaker or can be fed separately into the tubing system between beaker and cooling drum of the rotary die machine.

After manufacture of the encapsulation material, the liquid preparation is transported into the slit dies (left/right) by two heated gear pumps. Each slit die is individually fed by one pump. Upon pressure built up, the gellan melt is formed into ribbons which are being fed onto rotating casting drums. The inner surface of the slit dies is teflonized in order to avoid adherence of the melt to the slit die. Teflon also guarantees a more homogeneous melt flow inside the slit dies. The outlet port of the die is a slit; its width is defined by a spacer which is inserted between the two parts of the die. Different thicknesses of the spacers define the slit width, and therefore also the ribbon thickness. The slit dies are mounted close to the surface of the rotating cooling drums at constant height. The distance between slit die and casting drum can be adjusted as well. The ribbons are cooled down on conventional rotating casting drums which operate at a temperature of 50°C. The cooling is adjusted to gellan ribbons and is different to the cooling temperature of gelatine ribbon (18-22 °C).

Encapsulation is performed on a conventional Kamata rotary die machine (Jumbo). For encapsulation conventional rotary rolls are used. The rims of the die cups are typically somewhat higher, i.e. 0.75 mm, in contrast to 0.60 mm for standard gelatine preparations. In order to stabilize the capsules, they are pre-dried at ambient conditions on ventilated covered conveyer belt with a length of approx. 2 m. The belt operates at a rotational speed of approx. 0.02m/s. The pre-dried capsules are subsequently transferred via an intermediate tumbler into a conventional tumble dryer where the capsules are dried until they exhibit an appropriate moisture level, e.g. a residual water activity value a_{w} < 30 %. During the course of the drying procedure the capsules may be de-oiled.

### Example 2

### Capsule composition

Table 1 and 2 describe different formulations according to the present invention. While table 1 mentions the compositions of Batch No 1-4, excluding the purified water, table 2 lists the compositions including the purified water.

**Table 1**

| **Formulation excluding purified water** | | | | | |
|---|---|---|---|---|---|
| Batch No. | Kelcogel LT100 high acyl | Kelcogel F low acyl | Sodium citrate | Glycerol | Unipur GA (Starch) |
| | (%) | (%) | (%) | (%) | (%) |
| 1 | 6.73 | 0.46 | 0.19 | 28.85 | 63.77 |
| 2 | 6.62 | 0.23 | 0.19 | 30.26 | 62.71 |
| 3 | 6.62 | 0.23 | 0.19 | 30.26 | 62.71 |
| 4 | 6.52 | 0.00 | 0.19 | 29.78 | 63.51 |

**Table 2**

| **Formulation including purified water** | | | | | | |
|---|---|---|---|---|---|---|
| Batch No. | Kelcogel LT100 high acyl | Kelcogel F low acyl | Sodium citrate | Glycerol | Unipur GA (Starch) | Water purified |
| | (%) | (%) | (%) | (%) | (%) | (%) |
| 1 | 3.50 | 0.24 | 0.10 | 15.00 | 33.16 | 48.00 |
| 2 | 3.50 | 0.12 | 0.10 | 16.00 | 33.16 | 47.12 |
| 3 | 3.50 | 0.12 | 0.10 | 16.00 | 33.16 | 47.12 |
| 4 | 3.50 | 0.00 | 0.10 | 16.00 | 34.12 | 46.28 |

The tables outline the different formulas proposed with respect to the content of shell material at the weighing stage and upon addition of purified water during the preparation of wet gellan material used for encapsulation. The tables display the amount (%) of Kelcogel LT 100 high acyl, Kelcogel F low acyl, sodium citrate, glycerol, Unipur GA starch (table 1) as well as the water containing composition with the amount (%) of Kelcogel LT 100 high acyl, Kelcogel F low acyl, sodium citrate, glycerol, Unipur GA and purified water (table 2) which are used for processing.

Batch No. 1 contains 0.46 % of Kelcogel F low acyl, whereas preparation batch No. 4 comprises no Kelcogel F low acyl at all.

Batch No. 2 and 3 exhibit identical compositions but were processed in a way to obtain different shell and seam thicknesses by adjusting the ribbon thickness onto the casting drum of 0.40-0.45 mm for batch No 2 and 0.20-0.25 mm for batch No. 3, respectively. Batches No. 1 and 4 were executed with a ribbon thickness of 0.20-0.25 mm.

### Example 3

### In-Process-Data

The IPC data, obtained for gellan capsules before and after the drying process are summarized in table 3. Due to shrinkage upon loss of water during the drying process, the seam and shell thickness decrease.

**Table 3**

| IPC | | | | | | | | | | | | | | | | | | |
|---|---|---|---|---|---|---|---|---|---|---|---|---|---|---|---|---|---|---|
| Batch No. | Seam 1 before drying | | Seam 1 after drying | | Seam 2 before drying | | Seam 2 after drying | | Shell 1 before drying | | Shell 1 after drying | | Shell 2 before drying | | Shell 2 after drying | | Hardness after drying | |
| | N=10 | | N=10 | | N=10 | | N=10 | | N=10 | | N=10 | | N=10 | | N=10 | | N=10 | |
| | Ø (µm) | RSD (%) | Ø (µm) | RSD (%) | Ø (µm) | RSD (%) | Ø (µm) | RSD (%) | Ø (µm) | RSD (%) | Ø (µm) | RSD (%) | Ø (µm) | RSD (%) | Ø (µm) | RSD (%) | Ø (N) | RSD (%) |
| 1 | 201 | 15 | 199 | 24 | 166 | 10 | 154 | 18 | 255 | 8 | 238 | 7 | 214 | 11 | 121 | 6 | 5.8 | 2.2 |
| 2 | 436 | 11 | 292 | 21 | 405 | 12 | 235 | 26 | 601 | 5 | 368 | 14 | 535 | 5 | 316 | 10 | 5.8 | 2.5 |
| 3 | 271 | 6 | 179 | 12 | 247 | 11 | 158 | 9 | 348 | 8 | 254 | 15 | 286 | 9 | 186 | 18 | 5.4 | 3.8 |
| 4 | 243 | 10 | 175 | 20 | 195 | 11 | 146 | 14 | 273 | 32 | 214 | 13 | 258 | 8 | 164 | 14 | 5.7 | 1.7 |

Seam 1, seam 2, shell 1 and shell 2 refer to the mean seam/shell thickness as average value of N=10 individual thickness measurements. The measurement was performed by obtaining cross-sections of the capsules cut perpendicularly to the capsule's seam at the centre of the preparation. Seam 1 refers to the firstly (initially) formed seam during the rotary die process for each individual capsule; typically, this first seam is somewhat thicker than the seam formed upon closing of the capsule here denominated as second (final) seam, termed seam 2, established upon final closing of the capsule during processing.

Shell 1 and shell 2 describe the average thickness of the shell measured perpendicularly towards the established seam using the above cross-section. In the table given above shell 1 is the one which provides a somewhat higher average value in comparison to shell 2; the origin of the shell, i.e. from the left or from right ribbon, cannot be assigned.

### Example 4

### Disintegration behaviour

Table 4 describes the analytical data of the above formulations. It can be seen that preparation batch No. 1, 3, and 4 are not gastro-resistant, capsules from these batches disintegrate within 60 min applying EP and USP test conditions. Furthermore, capsules from batch 1, 3, and 4 pass USP rupture test showing capsule rupture within 15 min in water. Additionally, capsules from batch 1, 3, and 4 pass disintegration according to USP 701 showing capsule disintegration within 30 min.

The thicker preparation, i.e. batch No. 2 shows different behaviour, despite same shell formulation as batch No. 3. Due to the thicker seam and shell of batch No. 2 in comparison to Batch No. 1. Batch No. 3, and batch No. 4 these capsules doesn't comply to USP rupture test.

**Table 4**

| Batch No. | Disintegration | Disintegration | Rupture test | Disintegration |
|---|---|---|---|---|
| | EP 2.9.1 | USP 2040 | USP 2040 | USP 701 |
| | <60 min 0.1HCl | <60 min SGF | <15 min water | <30 min water |
| 1 | Capsules disintegrate | Capsules disintegrate | Complies | Complies |
| 2 | n.p.* | n.p.* | Does Not Comply | n.p.* |
| 3 | Capsules disintegrate | Capsules disintegrate | Complies | Complies |
| 4 | Capsules disintegrate | Capsules disintegrate | Complies | Complies |

| | | | | |
|---|---|---|---|---|
| * not performed | | | | |

### Example 5

### Disintegration behaviour determined with the texture analyzer

A texture analyzer (TAXT2i; Stable Micro Systems, Surrey, United Kingdom) was assembled with a disintegration rig to study the disintegration of gellan capsules. This mechanical test was designed to mimic the gastric disintegration conditions, while constantly maintaining the force and measuring the distance as the sample disintegrates. The apparatus was equipped with a 5 kg load cell and fitted with a 20 mm diameter cylindrical probe. The capsule was attached with a strip of 3 mm wide double-sided tape to the underside flat region of the probe end. Each capsule type was analyzed in duplicate. A double-jacketed glass vessel was connected with tubes to a thermostat system to keep the disintegration medium (FaSSGF=fasted state simulated gastric fluid from biorelevant.com, 100 ml) at 37 ± 0.5°C. On the bottom of the double-jacketed glass vessel is a platform, with a diameter of 30 mm, which was perforated to allow ingress of water beneath the capsule. The speed of the probe with the attached capsule was initially 2.0 mm/s until the surface of perforated platform was detected at the force of 0.029 N (threshold value for triggering the onset of texture analysis). Subsequently, the force of the probe was set to 0.2 N with a speed of 3.0 mm/s and the distance was measured to obtain the capsule's disintegration profile. The analyses were performed for 60 min in FaSSGF.

Two capsule batches were analysed:

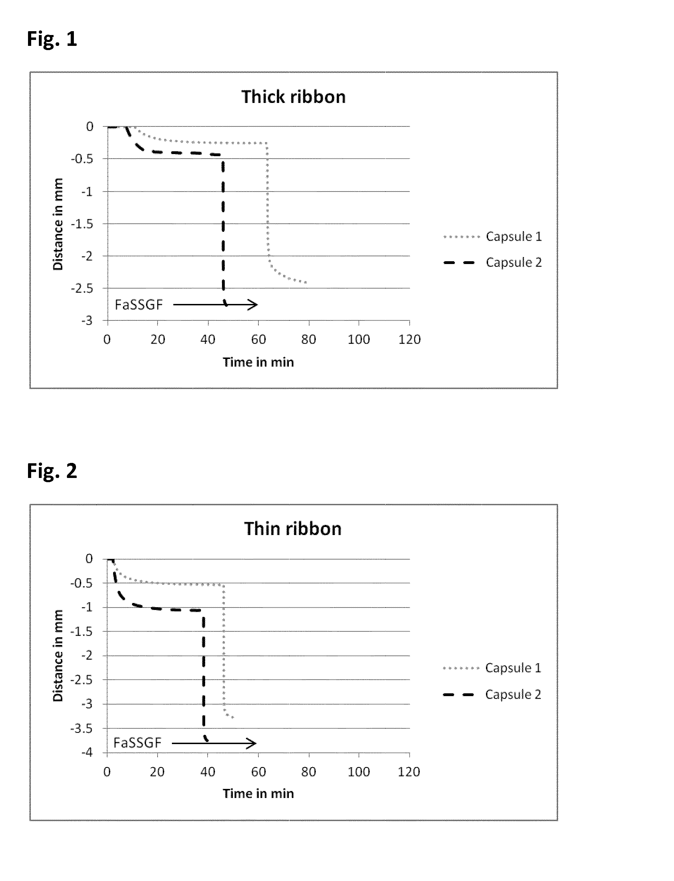

Fig. 1 shows Batch No. 2, thick ribbon, see example 2 for details on the formulation
Fig. 2 shows Batch No. 3, thin ribbon, see example 3 for details on the formulation

Disintegration profiles of gellan capsules were analyzed by texture analyzer in bio-relevant medium at 37 ± 0.5°C in FaSSGF, n=2.

Both gellan capsules from batch No. 2 and batch No. 3 showed no gastroresistance, since they were disintegrating in FaSSGF. Gellan capsules originating from the thick ribbon showed elongated disintegration time in comparison to capsules originating from thin ribbon. Therefore, non gastroresistance can, besides the shell composition, be adjusted on appropriate selection of the ribbon thickness during processing and as a consequence upon the thickness of seam and shell and its ratio.

## Claims

1. Process for manufacturing a gellan soft shell capsule, wherein
a) at least one starch is mixed with water to provide a homogeneous suspension,
b) at least one plasticizer is mixed with a high acyl gellan gum having more than 40 % acetyl and more than 45 % glyceryl residual substituents per repeat unit,
c) the suspension of a) is mixed with the mixture of b) providing a mixed suspension,
d) the mixed suspension of c) is heated to a temperature of 96°C to 99°C,
e) the heated suspension is transported to a encapsulation device in which a filling material is encapsulated by forming a shell with two seams to provide the soft shell capsules.

2. Process of claim 1,
**characterized in that** after step e) the soft shell capsules are dried, preferably by a pre-drying step on a ventilated conveyor belt and/or a drying step in a tumbler.

3. Process of any of claims 1 or 2,
**characterized in that** in step b) also a low acyl gellan gum having less than 25 % acetyl and less than 15 % glyceryl residual substituents per repeat unit is added.

4. Process of any of claims 1 to 3,
**characterized in that** the encapsulation device is a rotary die machine having slit dies, which are preferably teflonized, wherein the width of the slit dies is adjusted by a spacer to impact the thickness of the shell and the seams of the capsule.

5. Process of any of the preceding claims,
**characterized in that** the at least one starch is a native or a modified starch, preferably selected from the group consisting of potato starch, mung bean starch, corn starch, sago starch, tapioca starch, waxy starch, pea starch and its mixtures, wherein the modification can be physically, chemically or by hydrolysis.

6. Process of any of the preceding claims,
**characterized in that** the at least one plasticizer is selected from the group consisting of glycerol, xylitol, sorbitol, polyglycerol, non-crystallising solutions of sorbitol, glucose, fructose, glucose syrup, sorbitol/sorbitan solutions, propylene glycol, polyethylene glycols with low molecular weight and combinations thereof, wherein the plasticizer has preferably a water content of less than 17.5 % (w/w), more preferably of less than 2.5 % (w/w).

7. Process of any of the preceding claims,
**characterized in that** in/after step b) also an agent is added selected from the group consisting of coloring agents, opacifying agents, antioxidants, preservatives sweeteners, flavoring agents and its mixtures.

8. Process of any of the preceding claims,
**characterized in that** that soft shell capsule is filled with a filling material selected from the group consisting of foods, flavourings, vitamins, pharmaceuticals, detergents, liquids, semi-solids, suspensions, cosmetics, bath oils and its mixtures.

9. Soft shell capsule with a filling material encapsulated in a capsule shell producible by the process according to any of the preceding claims.

10. Soft shell capsule of claim 9,
**characterized in that** the capsule does not comply with the requirements as defined for capsules with gastric resistant properties, i.e. the capsule is not gastro resistant according to the USP <2040> and disintegrates within 120 min, preferably within 60 min using simulated gastric fluid as the immersion fluid and/or the capsule is not gastro resistant according to EP test monograph 2.9.1 using 0.1 M hydrochloric acid as the immersion fluid, and disintegrates within 120 min, preferably within 60 min.

11. Soft shell capsule according to any of claims 9 or 10,
**characterized in that** the capsule disintegrates according to USP <701> using water or 0.1 M hydrochloric acid or artificial gastric juice R as the immersion fluid within 120 min, preferably within 60 minutes and even more preferably within 30 minutes.

12. Soft shell capsule according to any of claims 9 to 11,
**characterized in that** the capsule disintegrates according to EP test monograph 2.9.1 using water or 0.1 M hydrochloric acid or artificial gastric juice R as the immersion fluid within 120 min, preferably within 60 minutes and even more preferably within 30 minutes.

13. Soft shell capsule according to any of claims 9 to 12,
**characterized in that** the capsule ruptures within 15 min according to USP <2040> using water as the immersion fluid.

14. Soft shell capsule of any of claims 9 or 13,
**characterized in that** the capsule has a mean hardness after drying of 2 to 12 N, preferably 3 to 10 N and more preferably 4 to 8 N.

15. Soft shell capsule of any of claims 9 to 14,
**characterized in that** the shell has a thickness after drying of 100 to 900 µm, preferably 150 to 700 µm and more preferably 200 to 500 µm.

16. Soft shell capsule of any of claims 9 to 15,
**characterized in that** the shell comprises a first and a second seam, wherein the first seam has a thickness after drying of 50 to 700 µm, preferably 60 to 600 µm and more preferably 100 to 500 µm and the second seam has a thickness after drying of 55 to 650 µm, preferably 65 to 550 µm and more preferably 105 to 505 µm.

17. Soft shell capsule of any of claims 9 to 16,
**characterized in that** the capsule shell has the following composition:
5 to 8 % (w/w), preferably 6 to 7 % (w/w) high acyl gellan gum,
0 to 3 % (w/w), preferably 0.1 to 2.0 % (w/w) low acyl gellan,
50 to 70 % (w/w), preferably 60 to 65 % (w/w) of the starch, and
20 to 40 % (w/w), preferably 28 to 32 % (w/w) of the plasticizer.
